# EUROPÄISCHE PATENTSCHRIFT

(11) **EP 0 775 155 B1**
(45) Veröffentlichungstag und Bekanntmachung des Hinweises auf die Patenterteilung: **06.05.2004**
(21) Anmeldenummer: 95925679.3
(22) Anmeldetag: 03.07.1995
(51) Int. Cl.: C07J 41/00, A61K 31/56, C07J 53/00, C07J 43/00

(54) **ESTRA-1,3,5(10)-TRIEN-DERIVATE, VERFAHREN ZU IHRER HERSTELLUNG UND DIESE VERBINDUNGEN ENTHALTENDE PHARMAZEUTISCHE ZUSAMMENSETZUNGEN**
ESTRA-1,3,5(10)-TRIENE DERIVATIVES, METHODS OF PREPARING SUCH COMPOUNDS AND PHARMACEUTICAL COMPOSITIONS CONTAINING THEM
DERIVES ESTRA-1,3,5(10)-TRIENE, LEURS PROCEDES DE PREPARATION ET COMPOSITIONS PHARMACEUTIQUES LES CONTENANT

(30) Priorität: 09.08.1994 DE 4429397
(43) Veröffentlichungstag der Anmeldung: 28.05.1997
(60) Teilanmeldung: 02090252.4
(73) Patentinhaber: Schering AG, 13353 Berlin (DE)
(72) Erfinder: SIEMANN, Hans-Joachim, verstorben (DE); SCHWARZ, Sigfrid, D-07743 Jena (DE); ELGER, Walter, D-14195 Berlin (DE); REDDERSEN, Gudrun, D-07749 Jena (DE); SCHNEIDER, Birgitt, D-07745 Jena (DE)
(74) Vertreter: Ziebig, Marlene K., Dr.
(86) Internationale Anmeldenummer: PCT/DE1995/000877
(87) Internationale Veröffentlichungsnummer: WO 1996/005216

(56) Entgegenhaltungen:
- EP-A- 0 430 386
- WO-A-94/01450
- DD-A- 114 806
- DD-A- 201 143
- DD-A- 207 447
- DE-A- 1 949 095
- FR-A- 2 133 484
- FR-A- 2 429 797
- GB-A- 1 317 373
- GB-A- 1 398 026
- Z. CHEM. , Bd. 10, Nr. 8, 1970 Seiten 299-300, SCHWARZ S ET AL 'Steroids. XI. 17.alpha.-Ethynylestradiol sulfamates'
- PHARMAZIE, Bd. 30, Nr. 1, 1975 BERLIN DE, Seiten 17-21, SCHWARZ S ET AL 'Steroids. 15. Sulfonyloxy derivatives of estrogens'
- PHARMAZIE, Bd. 30, Nr. 1, 1975 BERLIN DE, Seiten 52-53, STOELZNER W ET AL 'Dissociation of the antigonadotrophic and the contraceptive activities of certain estratriene derivatives'
- CHEMICAL ABSTRACTS, vol. 091, no. 25, 17.Dezember 1979 Columbus, Ohio, US; abstract no. 204845, SHU H D ET AL 'Structure-activity relationships of estradiol derivatives' Seite 97; Spalte 2; & YAO HSUEH HSUEH PAO , Bd. 14, Nr. 6, 1979 1ST MED. COLL. SHANGHAI;DEP. PHARMACOL.; SHANGHAI; PEOP. R. CHINA, Seiten 343-348,
- JOURNAL OF MEDICINAL CHEMISTRY, Bd. 37, Nr. 2, 21.Januar 1994 WASHINGTON US, Seiten 219-221, HOWARTH N M ET AL 'Estrone sulfamates: potent inhibitors of estrone sulfatase with therapeutic potential'
- HELVETICA CHIMICA ACTA, Bd. 50, Nr. 1, 1967 BASEL CH, Seiten 281-288, J. KALVODA ET AL '7-alpha-Methylöstrogene'
- STEROIDS, Bd. 45, Nr. 3-4, 1985 SAN FRANCISCO US, Seiten 325-340, N. UBEROI ET AL 'Structure Activity Reltionships of some Unique Estrogens Related to Estradiol Are Predicted by Fit into DNA'
- JOURNAL OF MEDICINAL CHEMISTRY, Bd. 32, Nr. 7, 1989 WASHINGTON US, Seiten 1642-1652, R. PETERS ET AL '17-Desoxy Estrogen Analogues'
- STEROIDS: STRUCTURE, FUNCTION, AND REGULATION, Bd. 56, Nr. 4, 1991 MA US, Seiten 201-210, B. BHAVNANI ET AL 'Interaction of Ring-B Unsaturated Estrogens with Estrogen Receptors of Human Endometrium and Rat Uterus'

## Beschreibung

Die vorliegende Erfindung betrifft neue Estra-1,3,5(10) - trien-Amidosulfamate.

Estrogene spielen in der hormonalen Kontrazeption und in der klimakterischen Hormon-Replacement-Therapie (HRT) sowie bei der Behandlung gynäkologischer (z. B. Mammacarcinom) und andrologischer (z. B. Prostatacarcinom) Krankheitsbilder eine wesentliche Rolle.

In der HRT und für die Kontrazeption werden Estrogene ganz überwiegend in Kombination mit einem Gestagen eingesetzt, z. B. Levonorgestrel, Desogestrel, Gestoden, Drospirorenon, Norethisteron, Cyproteronazetat, Chlormadinonazetat, Dienogest.

Im Falle der Kontrazeption werden Estrogene dazu benötigt, um Follikelreifung und Ovulation sicher zu unterdrücken. Andererseits substituieren sie dann die weitgehend unterdrückte endogene, ovarielle Sekretion von Estradiol. Diese Substitution ist wesentlich für die Erhaltung eines artifiziellen Menstruationzyklus und anderer Funktionen der Sexualorgane, die mit einem Gestagen allein nicht befriedigend gelingt.

Daneben haben endogene Estrogene wichtige zentralnervöse und metabolische Funktionen im weiblichen Organismus.

Normale Estrogenspiegel tragen zum Wohlbefinden entscheidend bei (L. Zichella; Clinical Management of the Menopausal Woman; Int. J. of Fertil. and Menop. Studies, 38, Suppl. 1 (1993), 15-22). Ihre Anwesenheit wirkt dem Entstehen von Herz-Kreislauf-Erkrankungen über verschiedene Mechanismen entgegen, nämlich durch Erzeugung von "günstigen" Lipoproteinmustern im Blut (G. Samsioe; Hormone Replacement Therapy and Cardiovascular Disease; Int. J. of Fertil. and Menop. Studies, 38, Suppl. 1 (1993), 23-29), Hemmung der Lipideinlagerung in der Gefäßwand (T. B. Clarkson; Experimental Effects of Progesterone versus Progestins on Arterial Wall; Gynecol. Endocrinol., 6: Suppl. 1 (1992), 15), Senkung des Blutdrucks durch günstige Beeinflussung des Gefäßtonus (R. A. Lobo; Estrogen and Cardiovascular Disease; Ann. New York Acad. Sciences, 592 (1990), 286-294), Reduktion des Perfusionswiderstandes in wichtigen Gefäßgebieten, Dämpfung kontraktiler Reize am Gefäßmuskel (C. Jiang et al.; Acute effect of 17β-estradiol on rabbit coronary artery contractile responses to endothelin-1; Am. J. Physiol., 263 (1992), H271-H275). Unter der Wirkung von Estrogenen setzen die Gefäßinnenwände Faktoren (Prostacyclin) frei, die der Entstehung von Blutgerinnseln entgegen wirken.

Estrogene sind ferner bei der Frau zur Erhaltung der Knochenstruktur unerläßlich. Ihr Verlust kann die Entwicklung eines Knochenabbaus (Osteoporose) bewirken (C. Christiansen; Prevention and Treatment of Osteoporosis with Hormone Replacement Therapy; Int. J. of Fertil. and Menop. Studies, 38, Suppl. 1 (1993), 45-54). Die letztgenannten "zentralnervösen" und "metabolischen" Effekte der Estrogene sind ein wesentlicher Gesichtspunkt der HRT.

Bei allen positiven Aspekten der Estrogentherapie gibt es ungelöste Probleme, welche die therapeutische Anwendung von Estrogenen einschränken oder unerwünschte Wirkungen beinhalten.

Natürliche Estrogene (Estradiol, Estron, Estronsulfat, Ester von Estradiol, Estriol) werden bei oraler Anwendung nur zum geringsten Teil bioverfügbar (K. B. Lokind et al.; Oral bioavailability of 17β-estradiol and various ester prodrugs in the rat; Int. J. Pharmaceutics, 76 (1991), 177-182). Dieser Anteil ist individuell so variabel, daß generelle Dosisempfehlungen nicht möglich sind. Eine Verwendung natürlicher Estrogene (Estradiol) für hormonale Kontrazeption wurde wegen dieser pharmakokinetischen Gegebenheiten negativ beurteilt (W. Kuhnz et al.; Pharmacokinetics of Estradiol, Free and Total Estrone, in Young Women Following Single Intravenous and Oral Administration of 17β-Estradiol; Arzneimittel-Forschung/Drug Res., 43(II), 9, (1993), 966-973). Problematisch ist auch die rasche Eliminierung der Substanzen aus dem Blut. Die Estrogensubstitution in der HRT muß sehr oft individuell angepaßt werden. Die Entwicklung von Estradiolprodrugs mit dem Ziel, die orale Bioverfügbarkeit zu verbessern, führte zu negativen Ergebnissen (K. B. Lokind et al.; siehe oben).

Synthetische Estrogene weisen gleichfalls erhebliche Nachteile auf. Das wichtigste synthetisch abgewandelte estrogene Steroid ist das Ethinylestradiol (EE). Dieses Estrogen ist beherrschend in der oralen hormonalen Kontrazeption. Neben EE wird in wenigen Fällen das Mestranol eingesetzt, welches ein "Prodrug" ist und im Organismus zu EE verstoffwechselt wird (J. W. Goldzieher; Selected aspects of the pharmacokinetics and metabolism of ethinyl estrogens and their clinical implications; Am. J. Obstet. Gynecol., 163 (1990), 318-322). EE ist bei oraler Applikation (Mensch) viel besser bioverfügbar als die o. g. natürlichen Estrogene, allerdings variiert die orale Bioverfügbarkeit individuell außerordentlich stark. Goldzieher betonte unter pharmakodynamischen Gesichtspunkten die negative Bedeutung der Variation der Fläche unter der Kurve (AUC, area under the curve), der Halbwertszeit und der Zeit bis zu maximalen Blutspiegeln. Die höchste in dieser Studie gefundene AUC von 0-24 Stunden nach der Applikation war 2121 pg x h/ml. Der niedrigste AUC war 284 pg x h/ml. Eine ähnliche Streubreite der AUC um den Faktor 6 bis 7 ist auch in der Arbeit von Hümpel et al. beschrieben (M. Hümpel et al.; Comparison of Serum Ethinyl Estradiol, Sex-Hormone-Binding Globulin, Corticoid-Binding Globulin and Cortisol Levels in Women Using Two Low-Dose Combined Oral Contraceptives; Horm. Res., 33 (1990), 35-39).

Bei oraler Anwendung gelangen Wirkstoffe nach Resorption aus dem Darmlumen über die Leber in den Organismus. Für estrogene Wirkstoffe ist diese Tatsache von besonderer Bedeutung, da die Leber ein Erfolgsorgan für Estrogene ist und deren orale Gabe zu starken Estrogeneffekten in der Leber führt. Zu den Sekretionsaktivitäten, die in der menschlichen Leber durch Estrogene reguliert werden, gehören u. a. die Synthesen der Transportproteine CBG, SHBG, TBG, das Angiotensinogen, verschiedene Faktoren, die in der Physiologie der Blutgerinnung eine wichtige Rolle spielen und Lipoproteine.

Werden dem weiblichen Organismus natürliche Estrogene unter Umgehung der Leberpassage zugeführt, z. B. durch transdermale Applikation, so bleiben die genannten Leberfunktionen praktisch unverändert (U. Larsson-Cohn et al.; Some biochemical consequences of post-menopausal hormone replacement treatment; in: The Controversial Climacteric, Ed: P. A. van Keep et al.; MTP Press Ltd. (1982)). Therapeutisch äquivalente Dosen natürlicher Estrogene führen bei oraler Applikation zu deutlichen Reaktionen hepatischer Parameter: Anstieg von SHBG, CBG, Angiotensinogen, HDL (high density lipoproteins) (J. C. Stevenson et al.; Oral Versus Transdermal Hormone Replacement Therapy; Int. J. of Fertil. and Menop. Studies, 38, Suppl. 1 (1993), 30-35). Deutlich stärker ausgeprägt als bei natürlichen Estrogenen sind entsprechende hepatische Estrogeneffekte bei equinen Estrogenmischungen (sog. konjugierte Estrogene)(C. A. Mashchak et al.; Comparison of pharmacodynamic properties of various estrogen formulations; Am. J. Obstet. Gynecol., 144 (1982) 511-518). Noch stärkere hepatische Estrogenität besitzen das Ethinyl-Estradiol und das DES. Bezogen auf antigonadotrope Eigenschaften ist das EE in der Leber ca. 8-10 mal stärker estrogen wirksam als oral verabreichte natürliche Estrogene. Es liegt also eine sehr ungünstige Dissoziation von Eigenschaften vor (B. von Schoultz et al.; Estrogen Therapy and Liver Function - Metabolic Effects of Oral and Parenteral Administration; The Prostate, 14 (1989), 389-395).

Folgende Beobachtung zeigt, daß unerwünschte hepatische Estrogeneffekte nicht durch Dosisreduktion von EE in Kontrazeptiva vermeidbar sind. Die Reduktion von 30 µg auf 20 µg EE jeweils in Kombination mit 150 µg des gleichen Gestagens ergab nach 3 Monaten keine Reduktion der erheblich gestiegenen Angiotensinspiegel und allenfalls marginal reduzierte Werte nach 6 Monaten (A. Basdevant et al.; Hemostatic and metabolic effects of lowering the ethinylestradiol dose from 30 mcg to 20 mcg in oral contraceptives containing desogestrel; Contraception, 48 (1993), 193-204).

Im Falle der Estrogentherapie mit hoch dosierten Estrogenen bei Männern, die an Prostatacarcinom erkrankt sind, sind thromboembolische Komplikationen mit tödlichem Ausgang eine bekannte Komplikation (B. von Schoultz et al.; siehe oben).

In abgeschwächter Form bestimmt das Nebenwirkungspotential des EE in der Leber die Strategie der oralen hormonalen Kontrazeption.

Im Hinblick auf erwünschte kontrazeptive Effekte sowie die Erhaltung des monatlichen Menstruationsgeschehens einerseits und die Beachtung eines erheblichen Potentials von Nebenwirkungen andererseits, ist die schwere Steuerbarkeit der erwünschten Blutspiegel von EE ein großes Problem im Sinne einer Gratwanderung. Möglicherweise kann ein erheblicher Prozentsatz von Frauen orale Kontrazeptiva nicht anwenden, weil entweder Blutungsanomalien oder estrogenbedingte Nebenwirkungen die Akzeptanzgrenze überschreiten.

Unter hormonalen Kontrazeptiva steigt das Risiko, bestimmte kardiovaskuläre Erkrankungen zu erleiden und zu sterben, deutlich an (V. Wynn; Oral contraceptives and coronary disease; J. Reprod. Med., 36, Suppl. 3, (1991), 219-225). Da entsprechende Risiken altersabhängig sind (J. I. Mann; Oral contraceptives and myocardial infarction in young women; Pharmacol. steroid. Contracept. Drugs, Editors S. Garrattini und H. W . Berendes, Raven Press, New York, (1977), 289-296), haben verschiedene Gesundheitsbehörden davor gewarnt, hormonale Kontrazeptiva bei Frauen einzusetzen, die älter sind als 35 Jahre. Eklatante kardiovaskuläre Risiken bestehen bei Raucherinnen oberhalb von 35 Jahren, die hormonale Kontrazeptiva anwenden (F. A. Leidenberger; Klinische Endokrinologie für Frauenärzte, 382-383; J. I. Mann, siehe oben). Im Vergleich zu Kontrollpopulationen ist das Risiko tödlicher Kreislauferkrankungen bei Anwenderinnen oraler Kontrazeptiva um den Faktor 5 - 6 erhöht F.A. (Leidenberger, siehe oben). Diese Daten belegen, daß bei erheblichen Untergruppen geschlechtsreifer Frauen, herkömmliche hormonale Kontrazeptiva nicht oder nur mit unvertretbar hohem Risiko angewandt werden können.

Nach dem Stand der Wissenschaft ist die geschilderte Problematik dem Estrogenanteil in hormonalen Kontrazeptiva, nicht dem Gestagenanteil zuzuordnen (Skouby et al.; J. Obstet. Gynekol.; (1990), 1535-1537). Ein "Consens-meeting" kam zu der Feststellung, daß das Risiko von tödlchen Myokardinfarkten unabhängig von der Dauer der Anwendung besteht. Diese Feststellung belegt, daß die zum Tode führende Gerinnselbildung nicht etwa durch chronische Schädigungen der Arterienwände im Herzen erfolgt (Arteriosklerose), sondern durch akute Einflüsse auf Haemostasefunktionen in der Leber (R. A. Lobo, siehe oben). Die Reduktion von Estrogenwirkungen in der Leber erscheint daher geeignet, die geschilderten Risiken der hormonalen Kontrazeption und die geschilderten Anwendungsbeschränkungen zu eliminieren. Für natürliche Estrogene, d. h. Estrogene mit im Vergleich zu EE geringerer hepatischer Estrogenität, werden die für EE. beschriebenen Risiken ausdrücklich ausgeschlossen (R. A. Lobo, siehe oben).

Die HRT mit natürlichen Hormonen erfordert mit der heutigen Technologie durchweg individuelle Dosisanpassungen. Entsprechende Behandlungen sind mit großen Unsicherheiten behaftet und beinhalten konkret die Gefahr von Über- und Unterdosierung.

FR-2 133 484, GB-1 317 373, DD-114 806, DE-1 949 095, DD-201 143, DD-207 447, DB-1 398 026 und FR-2 429 797 sowie Schwarz et al., Pharmazie 30, (1975) 17-21, Stölzner et al., Pharmazie 30 (1975) 52-53 und Schwarz et al. Z. Chem. (1970) 229-300, beschreiben Estra-1,3,5(10)-trien-Derivate, welche an der OH-Gruppe in 3-Stellung eine N-substituierte Amidosulfonatgruppe tragen.

WO-94/01450 und EP-0 430 386 beschreiben 14,17-überbrückte 16-Hydroxyestratriene. Es sind keine Verbindungen offenbart, welche in der 3-Stellung Amidosulfonat-Gruppen tragen.

Howarth et al. beschreiben in J. Med. Chem. (1994) 37, 219-221, Estronsulfamate und deren therapeutisches Potential zur Hemmung Steroid-Sulfatase.

Kalvoda et al., Helvetica Chimica Acta (1967) 50, 281-288, Uberoi et al., Steroids (1985) 45, 325-340, Peters et al., J. med. Chem. (1989) 32, 1642-1652, Bhavnani et al., Steroids (1991), 201-210 und Chemical Abstracts Band 91, Seite 97, Nr. 204845 t (1979), beschreiben Estra-1,3,5(10)-trien-Derivate, welche an der 3-OH-Gruppe eine Ethersubstitution tragen. Zu diesen Verbindungen werden pharmakologische Wirkungen beschrieben.

Der vorliegenden Erfindung liegt die Aufgabe zugrunde, Estra-1,3,5(10)-trien-Derivate zu schaffen, welche ein gutes Verhältnis von estrogener zu hepatischer Wirkung aufweisen.

Erfindungsgemäß wird die Aufgabe dadurch gelöst, daß Estra-1,3,5(10)-trien-Derivate der allgemeinen Formel I 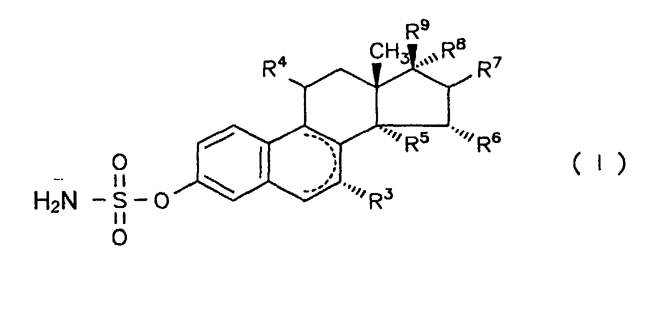 zur Verfügung gestellt werden,
worin
R³ ein Wasserstoffatom oder eine Alkylgrupe mit 1-5 C-Atomen ist,
R⁴ ein Wasserstoffatom, eine Hydroxygruppe, eine veresterte Hydroxygruppe, eine Halogenalkylgruppe mit 1-5 C-Atomen oder eine Alkoxygruppe mit 1-5 C-Atomen darstellt,
R⁵ und R⁶ jeweils ein Wasserstoffatom oder zusammen eine Methylengruppe bedeuten,
R⁷, R⁸ und R⁹ unabhängig voneinander ein Wasserstoffatom oder eine Hydroxygruppe darstellen, wobei diese Hydroxygruppe verestert oder verethert sein kann, und der Ring B gegebenenfalls eine oder zwei Doppelbindungen enthält
oder gegebenenfalls
R⁸ ein Alkinylrest mit bis zu 5 Kohlenstoffatomen ist oder
R⁸ und R⁹ zusammen ein Sauerstoffatom darstellen oder
R⁵ und R⁸ eine Vinylen- oder Ethylengruppe darstellen, wobei 17-Oxo-estra-1,3,5(10)-trien-3-yl-amidosulfonat ausgenommen ist.

Die erfindungsgemäßen Estra-1,3,5(10)-trien-Derivate können gegebenenfalls weitere Doppelbindungen zwischen den C-Atomen 9 und 11, 8 und 14, 14 und 15 und/oder 15 und 16 enthalten.

Die erfindungsgemäßen Estra-1,3,5(10)-trien-Derivate können gegebenenfalls Oxogruppen an den C-Atomen 6, 7, 11, 15 und/oder 16 tragen.

Die erfindungsgemäßen Estra-1,3,5(10)-trien-Derivate können weitere Hydroxygruppen an den C-Atomen 6, 7, 9, und/oder 14 tragen, wobei diese Hydroxygruppen gegebenenfalls verestert oder verethert sein können.

Die Veresterung der Hydroxygruppen erfolgt mittels üblicher Derivate physiologisch verträglicher anorganischer und organischer Säuren. Diese sind beispielsweise Phosphorsäure, Schwefelsäure, Oxalsäure, Maleinsäure, Fumarsäure, Milchsäure, Weinsäure, Äpfelsäure, Citronensäure, Salicylsäure, Valeriansäure, Adipinsäure und Benzoesäure. Weitere verwendbare Säuren sind beispielsweise in Fortschritte der Arzneimittelforschung, Bd. 10, Seiten 224-225, Birkhäuser Verlag, Basel und Stuttgart, 1966, und Journal of Pharmaceutical Sciences, Bd. 66, Seiten 1-5 (1977) beschrieben.
Die Veretherung erfolgt mittels üblicher Derivate aliphatischer Alkohole mit bis zu 6 Kohlenstoffatomen.

Erfindungsgemäße 3-Amidosulfonat-Estra-1,3,5(10)-trien-Derivate sind vorzugsweise
17β-Hydroxy-14α,15α-methylen-estra-1,3,5(10)-trien-3-yl amidosulfonat,
17β-Hydroxy-19-nor-17α-pregn-1,3,5(10)-trien-20-in-3-yl amidosulfonat,
17α-Hydroxy-14α,15α-methylen-estra-1,3,5(10)-8-tetraen-3-yl amidosulfonat,
11β-Methoxy-17-oxo-estra-1,3,5(10)-trien-3-yl amidosulfonat,
17β-Hydroxy-estra-1,3,5(10)-trien-3-yl amidosulfonat, 17β-Hydroxy-estra-1,3,5(10),6,8-pentaen-3-yl amidosulfonat,
17α-Hydroxy-estra-1,3,5(10)-trien-3-yl amidosulfonat, 16α, 17β-Dihydroxy-estra-1,3,5(10)-trien-3-yl amidosulfonat,
11β-Chlormethoxy-17β-hydroxy-estra-1,3,5(10)-trien-3-yl amidosulfonat,
17β-Hydroxy-14α, 17α-vinylen-estra-1,3,5(10)-trien-3-yl amidosulfonat,
16α,17β-Dihydroxy-14α,17α-ethylen-estra-1,3,5(10)-trien-3-yl amidosulfonat,
17β-Hydroxy-7α-methyl-estra-1,3,5(10)-trien-3,11β-diyl 3-Amidosulfonat-11-nitrat, 17β-Hydroxy-11β-methoxy-19-nor-17α-pregn-1,3,5(10)-trien-20-in-3-yl amidosulfonat.

Besonders bevorzugt sind ferner Estra-1,3,5(10)-trien-Derivate der allgemeinen Formel I, wobei R⁷ und R⁹ Hydroxygruppen bedeuten.

Besonders bevorzugt sind Estra-1,3,5(10)-trien-Derivate der allgemeinen Formel I, wobei R⁵ und R⁶ zusammen eine Methylengruppe darstellen.

Gegenstand der vorliegenden Erfindung ist ferner ein Verfahren zur Herstellung der erfindungsgemäßen Estra-1,3,5(10)-trien-Derivate, welches sich dadurch auszeichnet, daß man in an sich bekannter Weise ein Estra-1,3,5(10)-trien-Derivat der allgemeinen Formel (I) mit der oben angegebenen Bedeutung, mit der Maßgabe, daß in 3-Stellung eine OH-Gruppe vorhanden ist, mit einem entsprechend N-unsubstituierten Amidosulfonylchlorid unter Veresterung der 3-OH-Gruppe des Estra-1,3,5(10)-trien-Derivats umsetzt.

Die Umsetzung erfolgt üblicherweise in einem 2-Phasensystem in Gegenwart eines quartären Ammoniumsalzes als Phasen-Transfer-Katalysator. Die Umsetzung erfolgt bei Temperaturen zwischen Raumtemperatur und 100°C. Als Lösungsmittel werden übliche 2-Pasen-Systeme, wie Chloroform-Wasser, Dichlormethan-Wasser, Toluol-Wasser u.a. verwendet.

Ein weiterer Gegenstand der vorliegenden Erfindung sind pharmazeutische Zusammensetzungen, die als Wirkstoff Estra-1,3,5(10)-trien-Derivate der allgemeinen Formel I enthalten, wobei diese Zusammensetzungen gegebenenfalls geeignete Hilfs- und Trägerstoffe enthalten.

Die erfindungsgemäßen pharmazeutischen Zusammensetzungen enthalten gegebenenfalls zusätzlich eines oder mehrere der bereits erwähnten Gestagene, wie z. B. Levonorgestrel, Desogestrel, Gestoden, Drospirorenon, Norethisteron, Cyproteronazetat, Chlormadinonazetat, Dienogest.

Ferner können die erfindungsgemäßen pharmazeutischen Zusammensetzungen in Form von Mehrstufen- oder Kombinationspräparaten vorliegen.

Das Kombinationspräparat zur Kontrazeption besteht beispielsweise aus einer ersten Stufe, die eine Kombination mehrerer Komponenten, nämlich einem biogenen Estrogen, einem synthetischen Estrogen, einem Gestagen und/oder einem erfindungsgemäßen Estra-1,3,5(10)-trien-Derivat sowie gegebenenfalls einer oder mehreren weiteren Stufen, die aus einem pharmazeutisch unbedenklichen Placebo oder einem biogenen oder synthetischen Gestagen oder einem biogenen oder synthetischen Estrogen oder einem erfindungsgemäßen Estra-1,3,5(10)-trien-Derivat oder aus einer Kombination aus mehreren Komponenten, nämlich einem biogenen Estrogen, einem synthetischen Estrogen, einem Gestagen, einem erfindungsgemäßen Estra-1,3,5(10)-trien-Derivat oder einer Kombination aus synthetischen Estrogenen oder einem erfindungsgemäßen Estra-1,3,5(10)-trien-Derivat und einem Gestagen bestehen.

Das biogene Estrogen weist beispielsweise einen Bestandteil aus der Gruppe Estradiol, Estron, Estran, Estriol und anderen biogenen Estrogenen oder mindestens eine Verbindung, die einen der vorgenannten Estrogenbestandteile nach Einnahme schnell abspaltet, auf.

Das synthetische Estrogen weist erfindungsgemäß mindestens einen Bestandteil aus der Gruppe Ethinylestradiol, Mestranol und anderen synthetischen Estrogenen oder mindestens eine Verbindung, die einen der vorgenannten Estrogenbestandteile nach Einnahme schnell abspaltet, auf.

Das Gestagen weist erfindungsgemäß mindestens einen Bestandteil aus der Gruppe Levonorgestrel, Desogestrel, Progesteron, Norethisteronacetat, Chlormadinonacetat, Gestoden, Cyproteronacetat und anderen natürlichen und/oder synthetischen Gestagenen oder mindestens eine Verbindung, die einen der vorgenannten Gestagenbestandteile nach Einnahme schnell abspaltet, auf.

Ein weiterer Gegenstand der vorliegenden Erfindung ist es, pharmazeutische Zusammensetzungen zur Verfügung zu stellen, welche zur hormonalen Kontrazeption, klimakterischen Hormonsubstitutions-Therapie und zur Behandlung gynäkologischer und andrologischer Krankheitsbilder wie Mammacarinomen und Prostatacarcinomen eingesetzt werden können.

Ein weiterer Gegenstand der vorliegenden Erfindung sind pharmazeutische Zusammensetzungen in Form von Tabletten, Tabletten mit kontrollierter Freisetzung, Dragees, Pillen, Kapseln, Filmtabletten und Filmtabletten mit kontrollierter Freisetzung.

Die Arzneimittel der Erfindung werden mit den üblichen festen oder flüssigen Trägerstoffen oder Verdünnungsmitteln und den üblicherweise verwendeten pharmazeutischtechnischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver oder Depotformen.

Entsprechende Tabletten können beispielsweise durch Mischen des Wirkstoffes mit bekannten Hilfsstoffen, beispielsweise inerten Verdünnungsmitteln wie Dextrose, Zukker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Wegen der schwerwiegenden Nachteile der herkömmlichen auf medizinischem Gebiet eingesetzten Estrogenderivate besteht jedoch ein dringendes Bedürfnis nach Verbindungen, welche die oben genannten Nachteile nicht aufweisen.

Überraschenderweise wurde gefunden, daß die erfindungsgemäßen Verbindungen, welche EE hinsichtlich estrogener Wirksamkeit übertreffen, aber bei maximalen genitalen Estrogeneffekten im Uterus, hepatisch nicht stärker estrogen sind als das natürliche Estrogen Estradiol. Durch diese Konstellation werden mit den erfindungsgemäßen Verbindungen die therapeutischen Eigenschaften im Vergleich zu natürlichen und synthetischen Estrogenen entscheidend verbessert.

Kontrazeptiva, welche die erfindungsgemäßen Estra-1,3,5(10)-trien-Derivate enthalten, sind geeignet, Anwendungsbeschränkungen für hormonale Kontrazeption völlig neu zu definieren, da sie weniger oder keine Wirkung auf das Haemostasesystem haben.

Kontrazeptiva, welche die erfindungsgemäßen Estra-1,3,5(10)-trien-Derivate enthalten, können wegen dramatisch gesenkter Estrogeneffekte so hoch dosiert werden, daß auch eine bessere Zykluskontrolle als mit herkömmlichen EE-Kontrazeptiva möglich ist.

Die Anwendung von EE für die Hormonsubstituions-Therapie wird wegen der Nebenwirkungsproblematik z. Z. strikt abgelehnt. Mit den erfindungsgemäßen Estra-1,3,5(10)-trien-Derivaten bestehen die für die nicht natürlichen (biogenen) Estrogene bestehenden Risiken nicht mehr. Im Vergleich zu den heute in der Hormonsubstituions-Therapie dominierenden natürlichen Estrogenen besthet der Vorteil einer weit überlegenen Steuerbarkeit, da die orale Bioverfügbarkeit definiert ist und nicht wie bei den biogenen Estrogenen individuell stark unterschiedlich ist.

Der Nachweis der hepatischen Estrogenität erfolgte an ovariektomierten Ratten. Die Versuchstiere, adulte weibliche Ratten (Züchter: HSD/WIN:WU), werden ovariektomiert (Tag -14). Zwei Wochen später beginnt die Behandlung durch 1 mal tägliche orale Applikation der jeweiligen Testsubstanzen.

Die Zuordnung der einzelnen Tiere zu ihren Gruppen erfolgte durch Randomisierung. Der Versuch wurde als Blockversuch durchgeführt. Die Tiere wurden vor Versuchsbeginn und bei Versuchsende gewogen.

Behandlungsbeginn ist definiert als Tag 1(=d1), Behandlungsende ist Tag 7 (=d7), am Tag 8 wurden die Tiere getötet, verschiedene Organe (Uteri, Nebennieren, Leber) entnommen, gewogen und für weitere Untersuchungen tiefgekühlt (-196°C) weitergegeben.

Blut wurde in Ethernarkose vor der Behandlung (d0) bzw. (d4) und (d8) aus dem retrobulbären Plexus entnommen. Im gewonnenen Serum wurden IGF₁, Angiotensin I, Cholesterin und HDL-Cholesterin bestimmt.

### Bestimmungsmethoden:

IGF₁ - RIA Firma bioMérieux;
Angiotensin - Modifizierter RIA für Reninaktivität, Firma Sorin;
Cholesterin/HDL - enzymatische Tests, photometrische Bestimmung, Reagentien Firma Dr. Bruno Lange GmbH.

Die Ergebnisse der Untersuchungen sind in Tabelle 1 dargestellt.

Die erfindungsgemäßen Estra-1,3,5(10)-trien-Derivate entsprechen bei oraler Applikation der uterinen Wirksamkeit von Ethinylestradiol (EE) oder übertreffen dieses in der Wirksamkeit. Gleichzeitig werden die Parameter der hepatischen Estrogenität nicht oder signifikant geringer beeinflußt als bei vergleichbaren Dosierungen von Ethinylestradiol (EE). Ferner ist zu beobachten, daß die Blutspiegel der erfindungsgemäßen Estra-1,3,5(10)-trien-Derivate wesentlich höher sind als die der vergleichbaren Substanzen Estradiol (E2), Ethinylestradiol (EE) und Estriol (E3).

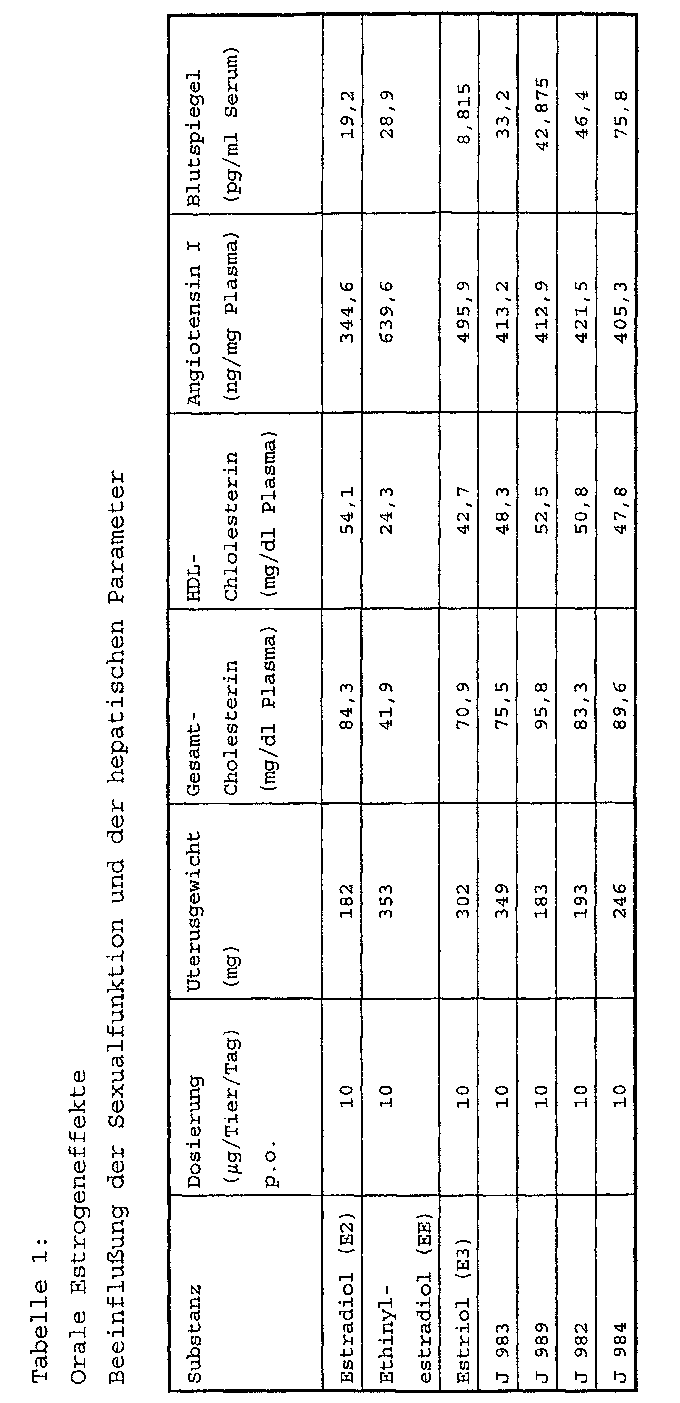

Die folgenden Beispiele erläutern die Erfindung:

### Beispiel 1:

Allgemeine Herstellungsvorschrift für N-unsubstituierte 3-Amidosulfonate von Estra-1,3,5-(10)-trien-Derivaten. Zu einer Lösung des Estra-1,3,5(10)-trien-Derivats in einem geeigneten Lösungsmittel (Dichlormethan, Pyridin oder Dimethylformamid) gibt man unter Rühren nacheinander eine Base (Triethylamin oder 2,6-Di-tert.butyl-4-methylpyridin) und das N-unsubstituierte Amidosulfonylchlorid. Hierbei soll die Reaktionstemperatur + 20°C nicht überschreiten. Nach 1 - 3 Stunden ist dünnschichtchromatographisch vollständiger Umsatz des Ausgangsmaterials festzustellen. Zur Aufarbeitung wird die Reaktionslösung mit verdünnter wäßriger Chlorwasserstoffsäure, mit gesättigter wäßriger Natriumhydrogencarbonatlösung und Wasser gewaschen, mit wasserfreiem Natriumsulfat getrocknet und im Vakkumrotationsverdampfer zur Trockene eingeengt. Der Rückstand wird durch Säulenchromatographie an Kieselgel und/oder Umkristallisation gereinigt.

### Beispiel 2 (= J 994): (Herstellungsbeispiel)

### Herstellung von Estron-Amidosulfonat

Estron (1 g) wird in Dimethylformamid (20 ml) gelöst. Zu dieser Lösung gibt man Amidosulfonylchlorid (2,14 g). Nach 6-stündigem Rühren wird mit Wasser gefällt und das Produkt aus Ethylacetat umkristallisiert, wobei man die Titelverbindung erhält.
Fp. 199 - 202°C, ¹H-NMR: 0,83 (s,H-18), 7,35(d,8,7 Hz, H-1), 7,9 (s, NH₂) ppm (D6-DMSO).

### Beispiel 3 (= J 995) :

### Herstellung von Estradiol-3-Amidosulfonat

Estron-Amidosulfonat (1,4 g) wird in einer Lösung aus Tetrahydrofuran (28 ml) und Methanol (28 ml) mit Natriumborhydrid (960 mg) reduziert. Nach Aufarbeitung wird das Rohprodukt aus Aceton umkristallisiert, wobei die Titelverbindung anfällt.
Fp. 211 - 213°C, ¹H-NMR: 0,67 (s,H-18), 3,53 (t,d 7,9 Hz, 4,7 Hz, H-17), 4,55 (d,4,8 Hz, OH), 7,34 (d, 8,6 Hz, H-1), 7,90 (s, NH₂) ppm (D6-DMSO).

### Beispiel 4 (= J 1018):

### Herstellung von 14α, 15α-Methylenestradiol-3-Amidosulfonat

Entsprechend Beispiel 1 bringt man 14α,15α-Methylenestradiol-17-tert.Butyldimethylsilylether (100 mg) in einer Lösung aus Dichlormethan (3 ml) und 2,6-Di-tert.butyl-4-methylpyridin (180 mg) mit Amidosulfonylchlorid (145 mg) zur Reaktion. Nach Aufarbeitung wird das Rohprodukt säulenchromatographisch gereinigt (Toluen/Aceton 4/1) und aus Aceton/n-Hexan umkristallisiert, wobei man 14α, 15α-Methylen-17β-tert.butyl-dimethylsilyloxy-estra-1,3,5(10)-trien-3-yl-Amidosulfonat erhält.
14α, 15α-Methylen-17β-tert.butyl-dimethylsilyloxy-estra-1,3,5(10)-trien-3-yl-Amidosulfonat (2,2 g) wird in Tetrahydrofuran (100 ml) gelöst. Zu dieser Lösung gibt man ein Gemisch aus Essigsäure/Wasser/Tetrahydrofuran 3/1/1 (220 ml). Die Reaktionslösung läßt man 7 Tage bei Raumtemperatur stehen, dann wird aufgearbeitet, das Produkt säulenchromatographisch gereinigt (Cyclohexan/Ethylacetat 3/2) und aus Aceton/n-Hexan umkristallisiert.
Fp. 210 - 214°C, ¹H-NMR: 0,20(m, CH₂), 0,26 (m, CH₂), 0,89 (s,H-18), 3,4 (m,H-17), 4,41 (d, 5,2 Hz, OH) 7,39 (d, 8,8 Hz,H-1), 7,90 (s, NH₂) ppm (D6-DMSO).

### Beispiel 5 (= J 1028) :

Herstellung von 17α-Ethinylestradiol-3-Amidosulfonat Entsprechend Beispiel 1 bringt man 17α-Ethinylestradiol-17-Trimethylsilylether (1,5 g) in einer Lösung aus Dichlormethan (40 ml) und Triethylamin (16 ml) mit Amidosulfonylchlorid (8 g) zur Reaktion. Nach Spaltung der Silylethergruppe und Aufarbeitung wird das Rohprodukt säulenchromatographisch gereinigt (Chloroform/Ethylacetat 7/3) und aus Aceton/n-Hexan umkristallisiert, wobei die Titelverbindung anfällt.
Fp. 209 - 211°C, ¹H-NMR: 0,76 (s, H-18), 3,35 (s, ≡CH), 5,35 (s, OH), 7,35 (d, 8,7 Hz, H-1), 7,89 (s, NH₂) ppm (D6-DMSO).

### Beispiel 6 (= J 1034):

### Herstellung von Estriol-3-Amidosulfonat

Entsprechend Beispiel 1 bringt man Estriol-16,17-Bis-tert.butyl-dimethylsilylether (2 g) in einer Lösung aus Dichlormethan (13 ml) und Triethylamin (15,5 ml) mit Amidosulfonylchlorid (7,9 g) zur Reaktion. Nach Aufarbeitung wird das Rohprodukt analog Beispiel 13 einer Silyletherspaltung unterworfen, die isolierte Substanz säulenchromatographisch gereinigt (Chloroform/Methanol/Essigsäure 90/13/1) und aus Aceton/n-Hexan umkristallisiert, wobei die Titelverbindung anfällt.
Fp. 208 - 213°C, ¹H-NMR: 0,67 (s, H-18), 3,30 (m, H-17), 3,84 (m, H-16), 4,7 (m,OH), 7,32 (d, 8,4 Hz, H-1) ppm (D6-DMSO).

### Beispiel 7 (= J 1050)

Herstellung von 17 α-Estradiol-3-Amidosulfonat Entsprechend Beispiel 1 bringt man 17 α-Estradiol-tert.butyl-dimethylsilylether (1,94 g) in einer Lösung aus Dichlormethan (70 ml) und 2,6-Di-tertbutyl-4-methylpyridin (3,6 g) mit Amidosulfonylchlorid (2,75 g) zur Reaktion. Nach Aufarbeitung wird das Rohprodukt säulenchromatographisch gereinigt (Toluen/Aceton 4/1) und aus Aceton/n-Hexan umkristallisiert. Das auf diese Weise erhaltene 17 α-tert.Butyl-dimethylsilyloxy-estra-1,3,5(10)-trien-3-yl-Amidosulfonat wird danach analog Beispiel 13 einer Silyletherspaltung unterworfen. Säulenchromatographie der isolierten Substanz (Toluen/Ethylacetat/Chloroform 6/3/1) und Umkristallisation aus Aceton/n-Hexan ergab die Titelverbindung.
Fp. 192 - 196°C, ¹H-NMR: 0,62 (s,H-18), 3,59 (d,5,5 Hz, H-17), 7,36 (d,8,8 Hz, H-1), 7,88 (s, NH₂) ppm (D6-DMSO).

### Beispiel 8 (= J 1010) :

Herstellung von 14 α, 15 α-Methylenestron-Amidosulfonat Entsprechend Beispiel 1 bringt man 14 α, 15 α-Methylenestron (765 mg) in einer Lösung aus Dichlormethan (50 ml) und Triethylamin (7,7 ml) mit Amidosulfonylchlorid (11,7 g) zur Reaktion. Nach Aufarbeitung wird das Rohprodukt säulenchromatographisch gereinigt (Chloroform/Ethylacetat 9/1) und aus Aceton/n-Hexan umkristallisiert, wobei man die Titelverbindung erhält.
Fp. 191 - 195°C, ¹H-NMR: -0,40 (m, CH₂), 0,80 (m, CH₂), 1,12 (s, H-18), 7,40 (d,8 Hz, H-1), 7,93 (s, NH₂) ppm (D6-DMSO).

### Beispiel 9 (= J 1021)

### Herstellung von 11 β-Methoxyestron-Amidosulfonat

Zu einer Lösung von 11 β-Methoxyestron (2 g) in Dimethylformamid (37 ml) gibt man portionsweise Natriumhydrid (0,4 g, 80 %). Nach Beendigung der Wasserstoffentwicklung fügt man Amidosulfonylchlorid (6,2 g) zu und rührt das Reaktionsgemisch bei Raumtemperatur über Nacht. Dann wird mit Wasser gefällt und das Produkt säulenchromatographisch gereinigt (Chloroform/Aceton 7/3). Umkristallisation aus Aceton/n-Hexan ergibt die Titelverbindung.
Fp. 191 - 195°C, ¹H-NMR: 0,99 (s, H-18), 3,20 (s, CH₃O), 4,24 (m, H11), 7,26 (d, 8,7 Hz, H-1), 7,93 (s, NH₂) ppm (D6-DMSO).

### Beispiel 10 (= J 1038)

### Herstellung von Estra-1,3,5(10)-trien-3, 17 β-diyl-3-amidosulfonat, 17-pentanoat

Estradiol-17-Pentanoat (2 g), gelöst in Dimethylformamid (37 ml), wird mit Natriumhydrid (336 mg, 80 %) und Amidosulfonylchlorid (6,47 g) zur Reaktion gebracht. Aufarbeitung, Säulenchromatographie (Chloroform/Ethylacetat 9/1) und Umkristallisation aus Aceton/n-Hexan führen zur Titelverbindung.
Fp. 107 - 108°C, ¹H-NMR: 0,78 (s, H-18), 0,87 (t,7,3 Hz, CH₃-(CH₂)₃-CO), 2,29 (t,7,2 Hz, C₃H₇-CH₂-CO),4,63 (dd,Σ 15,5 Hz, H-17), 7,34 (d, 8,4 Hz, H-1), 7,89 (s, NH₂)ppm (D6-DMSO).

### Beispiel 11 (= J 1051):

### Herstellung von 17 α-Hydroxy-14 α, 15 α-methylen-estra-1,3,5(10, 8-tetraen-3-yl-amidosulfonat

Entsprechend Beispiel 1 bringt man 14 α, 15 α-Methylen-17 α-trimethylsilyloxy-estra-1,3,5(10)-trien-3-ol (100 mg) in einer Lösung aus Dichlormethan (3 ml) und 2,6-Di-tert.butyl-4-methylpyridin (180 mg) mit Amidosulfonylchlorid (145 mg) zur Reaktion. Nach Spaltung der Silylethergruppe und Aufarbeitung wird das Rohprodukt säulenchromatographisch gereinigt (Cyclohexan/Ethylacetat 3/2) und aus Aceton/n-Hexan umkristallisiert.
Weißer Schaum, ¹H-NMR: 0,46 (m, CH₂), 0,92 (s,H-18), 1,28 (m,CH₂), 3,90 (d,Σ 6,0 Hz, H-17) ppm (CDCl₃), 7,35 (d, 8,8 Hz, H-1), 7,88 (s, NH) ppm (D6-DMSO).

### Beispiel 126 (= J 1054)

### Herstellung von Equilenin-Sulfamat

Equilenin (1 g) wird wie in Beispiel 2 beschrieben, mit Amidosulfonylchlorid in Dimethylformamid-Lösung verestert und aufgearbeitet.
Leicht gelbliches Harz, ¹H-NMR: 0,69(s,H-18), 7,23, 7,56(d, 8,4Hz, d, 8,5 Hz, H-6 und H-7), 7,82(d, 9,8Hz, H-1), 7,9(s, NH₂) ppm (D6-DMSO).

## Patentansprüche

1. Estra-1,3,5(10)-trien-Derivate der allgemeinen Formel I 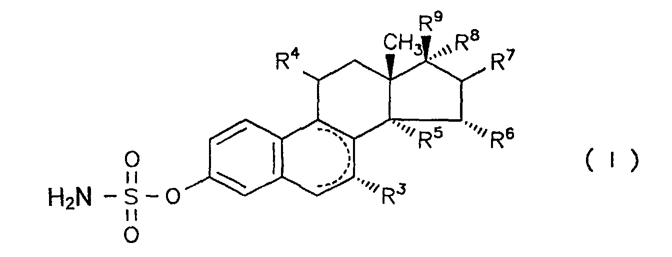 worin
R³ ein Wasserstoffatom oder eine Alkylgrupe mit 1-5 C-Atomen ist,
R⁴ ein Wasserstoffatom, eine Hydroxygruppe, eine veresterte Hydroxygruppe, eine Halogenalkylgruppe mit 1-5 C-Atomen oder eine Alkoxygruppe mit 1-5 C-Atomen darstellt,
R⁵ und R⁶ jeweils ein Wasserstoffatom oder zusammen eine Methylengruppe bedeuten,
R⁷, R⁸ und R⁹ unabhängig voneinander ein Wasserstoffatom oder eine Hydroxygruppe darstellen, wobei diese Hydroxygruppe verestert oder verethert sein kann,
zwischen den C-Atomen 9 und 11, 8 und 14, 14 und 15 und/oder 15 und 16 Doppelbindungen enthalten sein können,
Oxogruppen an den C-Atomen 6, 7, 11, 15 und/oder 16 befindlich sein können,
an den C-Atomen 6, 7, 9 und/oder 14 weitere Hydroxygruppen befindlich sein können, die verestert oder verethert sein können,
und der Ring B gegebenenfalls eine oder zwei Doppelbindungen enthält
oder gegebenenfalls
R⁸ ein Alkinylrest mit bis zu 5 Kohlenstoffatomen ist oder
R⁸ und R⁹ zusammen ein Sauerstoffatom darstellen oder
R⁵ und R⁸ eine Vinylen- oder Ethylengruppe darstellen,
wobei 17-Oxo-estra-1,3,5(10)-trien-3-yl-amidosulfonat ausgenommen ist.

2. Estra-1,3,5(10)-trien-Derivate gemäß Anspruch 1, nämlich
17β-Hydroxy-14α,15α-methylen-estra-1,3,5(10)-trien-3-yl amidosulfonat,
17β-Hydroxy-19-nor-17α-pregn-1,3,5(10)-trien-20-in-3-yl amidosulfonat,
17α-Hydroxy-14α,15α-methylen-estra-1,3,5(10)-8-tetraen-3-yl amidosulfonat,
11β-Methoxy-17-oxo-estra-1,3,5(10)-trien-3-yl amidosulfonat,
17β-Hydroxy-estra-1,3,5(10)-trien-3-yl amidosulfonat,
17β-Hydroxy-estra-1,3,5(10),6,8-pentaen-3-yl amidosulfonat,
17α-Hydroxy-estra-1,3,5(10)-trien-3-yl amidosulfonat,
16α, 17β-Dihydroxy-estra-1,3,5(10)-trien-3-yl amidosulfonat,
11β-Chlormethoxy-17β-hydroxy-estra-1,3,5(10)-trien-3-yl amidosulfonat,
17β-Hydroxy-14α, 17α-vinylen-estra-1,3,5(10)-trien-3-yl amidosulfonat,
16α,17β-Dihydroxy-14α,17α-ethylen-estra-1,3,5(10)-trien-3-yl amidosulfonat,
17β-Hydroxy-7α-methyl-estra-1,3,5(10)-trien-3,11β-diyl 3-Amidosulfonat-11-nitrat,
17β-Hydroxy-11β-methoxy-19-nor-17α-pregn-1,3,5(10)-trien-20-in-3-yl amidosulfonat.

3. Verfahren zur Herstellung der Estra-1,3,5(10)-trien-Derivate nach einem der Ansprüche 1 oder 2, **dadurch gekennzeichnet, daß** man in an sich bekannter Weise ein Estra-1,3,5(10)-trien-Derivat mit einem entsprechend substituierten Amidosulfonylchlorid unter Veresterung der 3-OH-Gruppe des Estra-1,3,5(10)-trien-Derivats umsetzt.

4. Pharmazeutische Zusammensetzungen enthaltend ein Estra-1,3,5(10)-trien-Derivat nach Anspruch 1 oder 2 gegebenfalls zusammen mit pharmazeutisch verträglichen Hilfs- und Trägerstoffen.

## Claims

1. Estra-1,3,5(10)-triene derivatives of general formula I 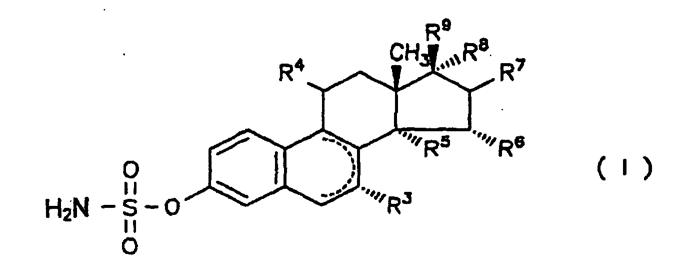 wherein
R³ is a hydrogen atom or an alkyl group having 1-5 C atoms,
R⁴ represents a hydrogen atom, a hydroxy group, an esterified hydroxy group, a haloalkyl group having 1-5 C atoms, or an alkoxy group having 1-5 C atoms,
R⁵ and R⁶ each represent a hydrogen atom or, when taken together, a methylene group,
R⁷, R⁸ and R⁹ each independently represent a hydrogen atom or a hydroxy group, said hydroxy group can be esterified or etherified,
double bonds can be included between the C atoms 9 and 11, 8 and 14, 14 and 15, and/or 15 and 16,
oxo groups can be present on the C atoms 6, 7, 11, 15, and/or 16,
further hydroxy groups can be present on the C atoms 6, 7, 9, and/or 14, which can be esterified or etherified, and ring B optionally includes one or two double bonds, or optionally,
R⁸ is an alkynyl residue having up to 5 carbon atoms, or
R⁸ and R⁹ together represent an oxygen atom, or
R⁵ and R⁸ represent a vinylene or ethylene group,
with 17-oxo-estra-1,3,5(10)-trien-3-yl amidosulfonate being excluded.

2. Estra-1,3,5(10)-triene derivatives according to claim 1, namely
17β-hydroxy-14α,15α-methylene-estra-1,3,5(10)-trien-3-yl amidosulfonate,
17β-hydroxy-19-nor-17α-pregna-1,3,5(10)-trien-20-yn-3-yl amidosulfonate,
17α-hydroxy-14α,15α-methylene-estra-1,3,5(10),8-tetraen-3-yl amidosulfonate,
11β-methoxy-17-oxo-estra-1,3,5(10)-trien-3-yl amidosulfonate,
17β-hydroxy-estra-1,3,5(10)-trien-3-yl amidosulfonate,
17β-hydroxy-estra-1,3,5(10),6,8-pentaen-3-yl amidosulfonate,
17α-hydroxy-estra-1,3,5(10)-trien-3-yl amidosulfonate,
16α,17β-dihydroxy-estra-1,3,5(10)-trien-3-yl amidosulfonate,
11β-chloromethoxy-17β-hydroxy-estra-1,3,5(10)-trien-3-yl amidosulfonate,
17β-hydroxy-14α,17α-vinylene-estra-1,3,5(10)-trien-3-yl amidosulfonate,
16α,17β-dihydroxy-14α,17α-ethylene-estra-1,3,5(10)-trien-3-yl amidosulfonate,
17β-hydroxy-7α-methyl-estra-1,3,5(10)-trien-3,11β-diyl 3-amidosulfonate 11-nitrate,
17β-hydroxy-11β-methoxy-19-nor-17α-pregna-1,3,5(10)-trien-20-yn-3-yl amidosulfonate.

3. A method of producing the estra-1,3,5(10)-triene derivatives according to any of claims 1 or 2, **characterized in that** an estra-1,3,5(10)-triene derivative is reacted in a *per se* known manner with an appropriately substituted amidosulfonyl chloride to esterify the 3-OH group of the estra-1,3,5(10)-triene derivative.

4. Pharmaceutical compositions, said compositions including an estra-1,3,5(10)-triene derivative according to claim 1 or 2, optionally together with pharmaceutically tolerable adjuvants and carriers.

## Revendications

1. Dérivés d'estra-1,3,5(10)-triène de formule générale I 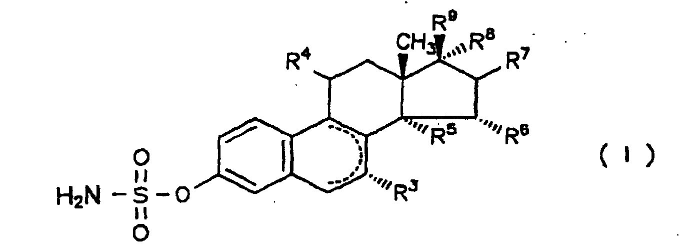 dans laquelle
R³ est un atome d'hydrogène ou un groupe alkyle comportant de 1 à 5 atomes de carbone,
R⁴ représente un atome d'hydrogène, un groupe hydroxy, un groupe hydroxy estérifié, un groupe halogénoalkyle comportant de 1 à 5 atomes de carbone ou un groupe alcoxy comportant de 1 à 5 atomes de carbone,
R⁵ et R⁶ représentent chacun un atome d'hydrogène ou ensemble un groupe méthylène,
R⁷, R⁸ et R⁹ représentent indépendamment l'un de l'autre un atome d'hydrogène ou un groupe hydroxy, ce groupe hydroxy pouvant être estérifié ou étherifié,
entre les atomes de carbone 9 et 11, 8 et 14, 14 et 15, et/ou 15 et 16, il peut se trouver des doubles liaisons,
des groupes oxo peuvent se trouver aux atomes de carbone 6, 7, 11, 15 et/ou 16, aux atomes de carbone 6, 7, 9 et/ou 14, il peut se trouver d'autres groupes hydroxy, qui peuvent être estérifiés ou éthérifiés,
et le cycle B contient éventuellement une ou deux doubles liaisons
ou éventuellement
R⁸ est un radical alkinyle comportant jusqu'à 5 atomes de carbone ou
R⁸ et R⁹ représentent ensemble un atome d'oxygène ou
R⁵ et R⁸ représentent un groupe vinylène ou éthylène,
en excluant le 17-oxo-estra-1,3,5(10)-trién-3-yle-amidosulfonate.

2. Dérivés d'estra-1,3,5(10)-triène selon la revendication 1, à savoir
17β-hydroxy-14α,15α-méthylén-estra-1,3,5(10)-trién-3-yle amidosulfonate,
17β-hydroxy-19-nor-17α-prégna-1,3,5(10)-trién-20-in-3-yle amidosulfonate,
17α-hydroxy-14α,15α-méthylén-estra-1,3,5(10),8-tétraén-3-yle amidosulfonate,
11β-méthoxy-17-oxo-estra-1,3,5(10)-trién-3-yle amidosulfonate,
17β-hydroxy-estra-1,3,5(10)-trién-3-yle amidosulfonate,
17β-hydroxy-estra-1,3,5(10),6,8-pentaén-3-yle amidosulfonate,
17α-hydroxy-estra-1,3,5(10)-trién-3-yle amidosulfonate,
16α,17β-dihydroxy-estra-1,3,5(10)-trién-3-yle amidosulfonate,
11β-chlorométhoxy-17β-hydroxy-estra-1,3,5(10)-trién-3-yle amidosulfonate,
17β-hydroxy-14α,17α-vinylén-estra-1,3,5(10)-trién-3-yle amidosulfonate,
16α,17β-dihydroxy-14α,17α-éthylén-estra-1,3,5(10)-trién-3-yle amidosulfonate,
17β-hydroxy-7α-méthyl-estra-1,3,5(10)-trién-3,11β-diyle 3-amidosulfonate-11-nitrate,
17β-hydroxy-11β-méthoxy-19-nor-17α-prégna-1,3,5(10)-trién-20-in-3-yle amidosulfonate.

3. Procédé de fabrication de dérivés d'estra-1,3,5(10)-triène selon une des revendications 1 ou 2, **caractérisé en ce qu'**on fait réagir de manière connue un dérivé d'estra-1,3,5(10)-triène avec un chlorure d'amidosulfonyle substitué de façon correspondante en estérifiant le groupe 3-OH du dérivé d'estra-1,3,5(10)-triène.

4. Compositions pharmaceutiques contenant un dérivé d'estra-1,3,5(10)-triène selon la revendication 1 ou 2, éventuellement en combinaison avec des adjuvants et des substances de support pharmaceutiquement compatibles.
